# EUROPÄISCHE PATENTANMELDUNG

(11) **EP 3 441 318 A2**
(43) Veröffentlichungstag der Anmeldung: **13.02.2019**
(21) Anmeldenummer: 18176910.0
(22) Anmeldetag: 11.06.2018
(51) Int. Cl.: B65B 59/04, B65B 3/00, B65B 3/04, B67C 3/02, F04B 43/08, B65B 39/00, B67C 3/28, F16K 7/06, F16K 7/07, A61M 5/168, A61M 39/28

(54) **FÜLLSTATION UND ABFÜLLANLAGE**

(30) Priorität: 08.08.2017 DE 102017213810
(71) Anmelder: Robert Bosch GmbH, 70442 Stuttgart (DE)
(72) Erfinder: Selig, Bertram, 74564 Crailsheim (DE); Stegmeier, Samuel, 74594 Wuestenau (DE); Duerner, Daniel, 91522 Ansbach (DE)

(57) **Zusammenfassung**

Die Erfindung betrifft eine Füllstation (1) zum Fördern von Füllgut von einem Reservoir (60) zu einer Ausgabe (80) durch einen Schlauch (4), aufweisend den Schlauch (4), eine den Schlauch (4) aufnehmende und austauschbare Fördervorrichtung (2), eine innerhalb eines Wellengehäuses (30) angeordnete Welle (31) und einen elektrischen Antrieb (40), wobei die Welle (31) mittels des elektrischen Antriebs (40) antreibbar ist und wobei die Welle (31) mit einem ersten Ende mit der Fördervorrichtung (2) verbindbar ist.

## Beschreibung

### Stand der Technik

Die vorliegende Erfindung betrifft eine Füllstation. Ferner betrifft die Erfindung eine Abfüllanlage.

Aus dem Stand der Technik ist eine Vielzahl von Füllstationen oder Kombifüllstationen bekannt. Aufgrund der Größe der Füllstationen wird ein erhöhter Bauraumbedarf innerhalb von Abfüllanlagen benötigt. Zudem gestaltet sich eine aseptische Schlauchverlegung innerhalb der Abfüllanlagen als schwierig und damit nachteilig für den Füll-/Dosierprozess. Nachteilig am Stand der Technik ist auch die meist aufwendige und komplexe Bauweise der Füllstationen.

### Offenbarung der Erfindung

Die Erfindung geht aus von einer Füllstation nach Gattung des unabhängigen Anspruchs 1. Ferner geht die Erfindung aus von einer Abfüllanlage nach Gattung des unabhängigen Anspruchs 10.

Die erfindungsgemäße Füllstation dient dem Fördern von Füllgut von einem Reservoir zu einer Ausgabe durch einen Schlauch. Die Füllstation weist den Schlauch, eine den Schlauch aufnehmende und austauschbare bzw. auswechselbare Fördervorrichtung, eine innerhalb eines Wellengehäuses angeordnete Welle und einen elektrischen Antrieb auf. Die Welle ist mittels des elektrischen Antriebs antreibbar und ist mit einem ersten Ende mit der Fördervorrichtung verbindbar ausgebildet. Dabei ist die Welle mit einem zweiten Ende mit dem elektrischen Antrieb verbunden.

Durch das Vorsehen der Füllstation lassen sich unterschiedlich ausgebildete Fördervorrichtungen auf das Wellengehäuse aufsetzen. Mittels der Welle ist eine vom elektrischen Antrieb erzeugte Drehbewegung vom elektrischen Antrieb auf die Hubvorrichtung auf einfache Weise übertragbar. Mittels des Wellengehäuses ist die Welle vor äußeren Einwirkungen auf einfache Weise geschützt. Vorzugsweise ist das Wellengehäuse eine Trägersäule.

Unter Füllgut können Stoffe oder Stoffgemische verstanden werden. Vorzugsweise ist Füllgut ein Gas oder eine Flüssigkeit, bevorzugt ein flüssiges pharmazeutisches Produkt.

Unter einer Füllstation kann eine Vorrichtung verstanden werden, die zum Abfüllen, Befüllen, Füllen oder Einbringen von Füllgut in Behälter oder Behältnisse eingerichtet ist. Vorzugsweise ist die Fördervorrichtung ein Adapterkopf oder ein Adapterkit für eine Füllstation.

Vorzugsweise ist ein erstes Ende des Schlauchs am Reservoir oder Füllgutreservoir (z.B. Tank) angeschlossen. Bevorzugt weist ein zweites Ende des Schlauchs eine Ausgabe auf, die insbesondere als Auslassöffnung oder Füllnadel ausgebildet ist. Besonders bevorzugt ist ein Abschnitt des Schlauchs zwischen dem erstem Ende und dem zweiten Ende durch die Fördervorrichtung geführt. Damit lässt sich Füllgut von der Fördervorrichtung in zu befüllende Behältnisse, die vorzugsweise auf einer Transportvorrichtung einer Abfüllanlage transportierbar sind, auf einfache Weise fördern.

Alternativ kann der Schlauch an seinem zweiten Ende an eine Drossel der Fördervorrichtung angeschlossen sein. Damit lässt sich Füllgut vom Füllgutreservoir auf einfache Weise in Richtung zur Fördervorrichtung fördern. Vorzugsweise ist ein erstes Ende eines zweiten Schlauches an die Drossel angeschlossen. Bevorzugt weist der zweite Schlauch an seinem zweiten Ende eine Ausgabe auf, die insbesondere als Auslassöffnung oder Füllnadel ausgebildet ist. Damit lässt sich Füllgut von der Fördervorrichtung in die zu befüllenden Behältnisse auf einfache Weise fördern.

Unter einem Schlauch kann ein elastischer Schlauch verstanden werden, insbesondere ein Silikonschlauch. Bevorzugt ist der Schlauch als ein Quetschschlauch ausgebildet. Vorzugsweise weist der Schlauch im vollquetschten Zustand keine Möglichkeit für das Füllgut auf, durch den Schlauch gefördert zu werden. Im Gegensatz hierzu kann das Füllgut im nicht gequetschten - also ungequetschten - Zustand des Schlauchs unbegrenzt durch den Schlauch gefördert werden. Bei einer nur teilweisen Quetschung des Schlauches kann eine Förderung von Füllgut reduziert werden. Unter einem Quetschen kann ein Zusammendrücken oder Zusammenpressen verstanden werden.

Vorzugsweise ist die Fördervorrichtung als ein Zeit-Druck-Füllsystem aufgebaut. Alternativ kann die Fördervorrichtung als eine Schlauchpumpe, eine Drehschieberpumpe, eine Drehkolbenpumpe, eine Rollmembranpumpe aufgebaut sein.

Die Unteransprüche zeigen bevorzugte Weiterbildungen der Erfindung.

Vorteilhaft weist die Fördervorrichtung zur Verbindung mit dem Wellengehäuse und zur Kopplung mit der Welle ein Schnellverschlussmittel auf. Bevorzugt ist das Schnellverschlussmittel als Adapter oder Adaptierung ausgebildet. Durch das Schnellverschlussmittel wird ein schnelles Verbinden, Austauchen, Auswechseln und/oder Ersetzen der Fördervorrichtung ermöglicht.

Dabei weist die Fördervorrichtung zusätzlich die Kupplung auf, insbesondere Motorkupplung, zur Verbindung mit der Welle. Durch das Vorsehen der Kupplung lässt sich bspw. eine Hubvorrichtung der Fördervorrichtung auf einfache Weise mit der Welle verbinden.

Weiter vorteilhaft weist die Fördervorrichtung eine mittels der Welle antreibbare Hubvorrichtung und eine klappbare Verschlusskappe auf. Mittels der klappbaren Verschlusskappe lässt sich eine verbesserte Zugänglichkeit zum Quetschlauch und den damit verbundenen Komponenten erzielen. Mit anderen Worten lässt sich der Schlauch und sowie weitere Bauteile der Fördervorrichtung im Bedarfsfall entnehmen und reinigen.

Vorzugsweise ist an der Verschlusskappe ein Hebel, eine Schwenkvorrichtung, Schiebervorrichtung oder ein Drehverschlussmittel angeordnet, welcher bzw. welches wenigstens teilweise durch die Verschlusskappe geführt sein kann. Hierdurch wird ein einfach ausführbares und sicheres Verschließen der Verschlusskappe ermöglicht.

Vorzugsweise ist die Verschlusskappe mittels eines Scharniers am Gehäuse der Fördervorrichtung verkippbar gelagert. Damit lässt sich mittels einer einzigen Hand die Verschlusskappe öffnen. Die Verschlusskappe kann als Deckel verstanden werden.

Weiter vorteilhaft weist die Hubvorrichtung ein Hubmittel wenigstens zur teilweisen oder vollständigen Quetschung des Schlauchs auf, wobei der Schlauch zwischen dem Hubmittel und der klappbaren Verschlusskappe der Fördervorrichtung angeordnet ist.

Vorzugsweise ist die Hubvorrichtung eingerichtet, bei einer Aktivierung des elektrischen Antriebs den Schlauch mittels des Hubmittels wenigstens teilweise oder vollständig zu quetschen. Der elektrische Antrieb kann ein Drehmoment erzeugen. Dieses Drehmoment wird auf die Hubvorrichtung übertragen. Die Hubvorrichtung, die vorzugsweise einen Gewindetrieb, insbesondere als Kugel-, Rollen- und/oder Planetenwälzgewindetrieb aufweist, wandelt eine Drehung, also das erzeugte Drehmoment des elektrischen Antriebs, in eine translatorische Bewegung um. Hierdurch ist das Hubmittel der Hubvorrichtung translatorisch verfahrbar. Mit anderen Worten erfährt das Hubmittel der Hubvorrichtung bei einer Aktivierung des elektrischen Antriebs eine lineare Verlagerung in eine Richtung hin zum Schlauch. Das Hubmittel verlässt dabei seine Ausgangsposition (keine Quetschung) bis es eine Endposition (teilweise oder vollständige Quetschung) erreicht hat.

Vorzugsweise ist die Hubvorrichtung eingerichtet, bei einer Aktivierung des elektrischen Antriebs eine den Schlauch quetschende Position wieder zu verlassen. Mit anderen Worten erfährt das Hubmittel der Hubvorrichtung bei der Aktivierung des elektrischen Antriebs eine lineare Verlagerung in eine Richtung weg vom Schlauch. Das Hubmittel verlässt dabei seine Ausgangsposition (teilweise oder vollständige Quetschung) bis eine Endposition (keine Quetschung) erreicht ist. Vorzugsweise verlagert sich das Hubmittel orthogonal zu einer Längsrichtung des Schlauchs.

Vorzugsweise ist die Hubvorrichtung damit eingerichtet, das Hubmittel von einer ersten Position in eine zweite Position zur Quetschung des Schlauchs zu verfahren und das Hubmittel von der zweiten Position zurück in die erste Position zur Beendigung der Quetschung des Schlauchs zu verfahren.

Vorzugsweise ist die Hubvorrichtung als ein Quetschventil oder eine Schlauchquetsche ausgebildet. Bevorzugt ist das Hubmittel ein mechanischer Stößel.

Bevorzugt ist die Hubvorrichtung mittels des elektrischen Antriebs antreibbar. Bevorzugt ist eine Welle zwischen der Hubvorrichtung und dem elektrischen Antrieb angeordnet.

Vorzugsweise weist das Hubmittel wenigstens ein erstes Quetschmittel auf. Bevorzugt ist der Schlauch zwischen dem ersten Quetschmittel und der klappbaren Verschlusskappe der Fördervorrichtung angeordnet bzw. positioniert.

Vorzugsweise weist das erste Quetschmittel eine Kante oder Fläche zur Quetschung des Schlauches auf. Bevorzugt ist das erste Quetschmittel als ein Keil ausgebildet. Besonders bevorzugt weist der Keil einen dreieckförmigen Querschnitt auf, wobei eine Grundfläche des Keils plan auf der Hubvorrichtung an.

Durch das Vorsehen der Verschlusskappe und dem ersten Quetschmittel lässt sich eine Quetschung des Schlauchs auf einfache Weise ausführen.

Bevorzugt weist die Verschlusskappe eine erste Erhöhung auf. Besonders bevorzugt ist die erste Erhöhung gegenüber vom ersten Quetschmittel verschlusskappenunterseitig angeordnet. Somit ist eine Quetschung des Schlauchs auf einfache Weise ausführen.

Weiter vorteilhaft weist die Hubvorrichtung einen Spindeltrieb zur Verlagerung des Hubmittels auf.

Durch das Vorsehen des Spindeltriebs kann die vom elektrischen Antrieb erzeugte Drehbewegung in eine Linearbewegung umgewandelt werden. Hierdurch ist eine axiale Verlagerung des Hubmittels mittels des Spindeltriebs erreichbar. Bevorzugt weist der Spindeltrieb eine Spindelmutter und eine Kugelumlaufspindel auf.

Weiter vorteilhaft weist die Fördervorrichtung ein Drosselventil auf, das an einer Seite des Schlauchs angeschlossen ist. Hierdurch kann eine Regulierung des Mengenstroms des verwendeten Füllguts erzielt werden.

Weiter vorteilhaft ist das Drosselventil mittels einer Haltevorrichtung an der klappbaren Verschlusskappe und am Gehäuse der Fördervorrichtung befestigbar. Hierdurch kann eine Fixierung des Drosselventils auf einfache Weise erzielt werden.

Besonders bevorzugt weist die Fördervorrichtung einen zweiten Schlauch auf, der an einem zweiten Ende des Drosselventils angeschlossen ist. Damit kann das Füllgut vom Reservoir zur Fördervorrichtung und weiter in zu befüllende Behältnisse gefördert werden. Dabei wird das Füllgut durch den Schlauch, die Drossel und den zweiten Schlauch gefördert.

Vorzugsweise kragt ein erster Teil der Haltevorrichtung an der Verschlusskappe aus, wobei ein zweiter Teil der Haltevorrichtung an einem Gehäuse der Fördervorrichtung angeordnet ist. Bevorzugt ist der erste Teil als wenigstens eine längliche Auskragung ausgebildet, die sich parallel zur Mittelachse des Drosselventils erstreckt. Besonders bevorzugt erstreckt sich der erste Teil wenigstens über einen ersten Teilbereich des Drosselventils. Vorzugsweise weist der zweite Teil eine Aussparung zur Aufnahme eines zweiten Teilbereichs des Drosselventils auf. Somit kann das Drosselventil zwischen den Teilen angeordnet und positioniert sein.

Weiter vorteilhaft weist die Fördervorrichtung einen Zwangsschließmechanismus auf.

Hierdurch wird eine zusätzliche Möglichkeit bereitgestellt, aktiv in den Förderprozess des Füllgutes durch den Schlauch eingreifen zu können und bei Bedarf eine Quetschung des Schlauches auszulösen. Vorzugsweise ist der Zwangsschließmechanismus als Ventil ausgebildet, insbesondere als Sicherungsventil.

Vorzugsweise weist der Zwangsschließmechanismus ein Hubmittel auf. Bevorzugt weist das Hubmittel wenigstens ein zweites Quetschmittel auf. Besonders bevorzugt ist der Schlauch zwischen dem zweiten Quetschmittel und der klappbaren Verschlusskappe der Fördervorrichtung angeordnet.

Vorzugsweise weist das zweite Mittel eine Kante oder Fläche zur Quetschung des Schlauches auf. Bevorzugt ist das zweite Quetschmittel als ein Keil ausgebildet. Besonders bevorzugt weist der Keil einen dreieckförmigen Querschnitt auf, wobei eine Grundfläche des Keils plan auf der Hubvorrichtung anliegt. Durch das Vorsehen der Verschlusskappe und dem zweite Quetschmittel lässt sich eine Quetschung des Schlauchs auf einfache Weise ausführen.

Bevorzugt weist die Verschlusskappe eine zweite Erhöhung auf. Besonders bevorzugt ist die zweite Erhöhung gegenüber vom zweiten Quetschmittel verschlusskappenunterseitig angeordnet. Somit ist eine Quetschung des Schlauchs auf einfache Weise ausführen.

Vorzugsweise ist der Zwangsschließmechanismus über einen Steuerluftanschluss mit Druckluft versorgbar.

Die erfindungsgemäße Füllstation kann als Kombifüllstation ausgebildet sein. Wenigstens eine der Füllstationen kann eine Kombifüllstation ausbilden. Vorzugsweise weist jede Füllstation jeweils einen elektrischen Antrieb, ein Wellengehäuse mit einer Welle und eine Fördervorrichtung auf.

Die erfindungsgemäße Abfüllanlage weist wenigstens eine Füllstation auf, wie oben beschrieben.

Hierdurch kann eine Mehrzahl an Füllstationen zum Fördern von Füllgut verwendet werden.

Vorzugsweise weist die Füllstation, ein Reservoir sowie eine Transportvorrichtung zum Transport zu befüllender Behältnisse auf. Bevorzugt sind die wenigstens eine Füllstation, das Reservoir und die Transportvorrichtung auf einer Grundplatte der Abfüllanlage angeordnet. Besonders bevorzugt ist an einer Unterseite der Grundplatte wenigstens ein elektrischer Antrieb angeordnet.

Durch das Vorsehen der Abfüllanlage ist eine verbesserte Schlauchverlegung innerhalb der Anlage ermöglicht, wobei unnötig lange Schläuche vermieden werden. Ferner stellt sich eine Erleichterung des aseptischen Aufrüstens ein und eine vereinfachte Reinigbarkeit.

Vorzugsweise weist ein Verfahren zum kontrollierten Fördern von Füllgut mittels wenigstens einer Füllstation die Schritte auf:
- Aktivieren des elektrischen Antriebs und im Ansprechen darauf
- Verlagern des Hubmittels der Hubvorrichtung von einer ersten Position in eine zweite Position und im Ansprechen darauf
- Wenigstens teilweises oder vollständiges Quetschen des Schlauchs mittels des Hubmittels.

Vorzugsweise ist das Verfahren ein Zeit-Druck-Füllverfahren. Bevorzugt erfolgt beim Zeit-Druck-Füllen die Füllmengendosierung bei einem nicht gequetschten Schlauch für eine zu bestimmende Zeit. Das Füllgut kann durch einen voreingestellten Druck beaufschlagt werden. Abhängig von Druck und Zeit und den verwendeten Formatteilen, wie Nadel-, Drossel-, Schlauch- Durchmesser, ergibt sich damit eine Füllmenge. Die Eigenschaften des Füllgutes sind zusätzlich abhängig von dessen Viskosität.

Vorzugsweise basiert die Füllmengendosierung, welche durch das gesteuerte Quetschen des Schlauchs erfolgt, auf einer internen Berechnung. Dem System ist aufgrund eines vorhergegangenen Einlernprozesses bekannt, welche Füllmenge abhängig vom vorhandenen Druck je Zeiteinheit durch die Fördervorrichtungen fließt. Bevorzugt wird beim Abfüllvorgang periodisch der aktuelle Druck im Verteilerrohr erfasst. Abhängig vom Druckverlauf während des Füllvorganges ergibt sich eine variable Öffnungszeit. Dadurch können noch während des Füllvorganges externe Einflüsse berücksichtigt werden. Damit ergibt sich letztendlich für jede einzelne Füllstelle eine genau passende Füllzeit zur Erzielung einer Sollfüllmenge. Diese Füllmengenkorrektur der einzelnen Füllstellen ergibt sich aus den von der In-Prozess-Kontrolle (IPK) für die Füllstelle gewonnenen Werten.

Bevorzugt weist das Verfahren zusätzlich die weiteren Schritte auf:
- Erneutes Aktivieren des elektrischen Antriebs und im Ansprechen darauf
- Verlagern des Hubmittels von der zweiten Position zurück in die erste Position zur Beendigung der wenigstens teilweisen oder vollständigen Quetschung des Schlauchs und im Ansprechen darauf
- Beenden der Quetschung des Schlauchs mittels des Hubmittels.

### Kurze Beschreibung der Zeichnungen

Nachfolgend werden Ausführungsbeispiele der Erfindung unter Bezugnahme auf die begleitende Zeichnung im Detail beschrieben. In der Zeichnung ist:
- Figur 1: eine schematische Ansicht einer Fördervorrichtung einer ersten erfindungsgemäßen Ausführungsform;
- Figur 2: einen schematischen Schnitt durch die Fördervorrichtung aus Figur 1;
- Figur 3: eine schematische Ansicht einer erfindungsgemäßen Ausführungsform einer Füllstation;
- Figur 4: eine weitere schematische Ansicht durch die Ausführungsform aus Figur 3;
- Figur 5: einen schematischen Schnitt durch die Ausführungsform aus Figur 3;
- Figur 6: eine schematische Ansicht einer erfindungsgemäßen Ausführungsform einer Abfüllanlage; und
- Figur 7: eine schematische Draufsicht auf die Ausführungsform aus Figur 6.

### Ausführungsformen der Erfindung

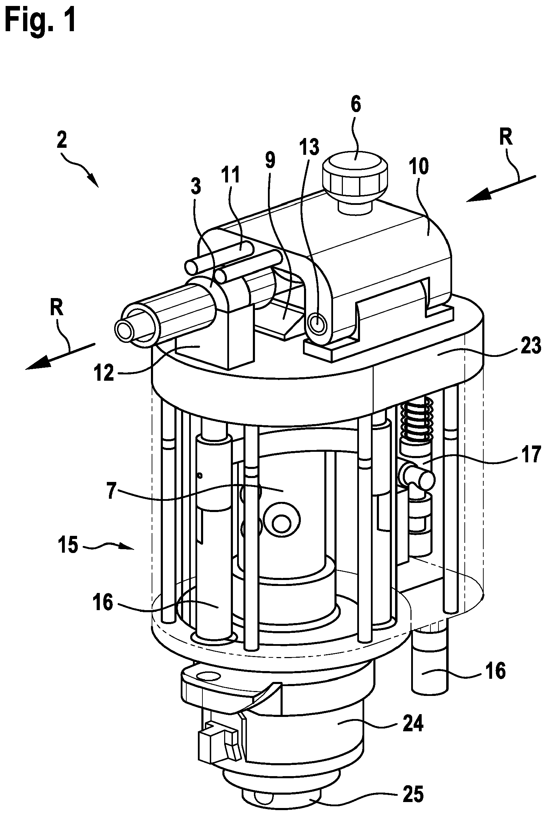

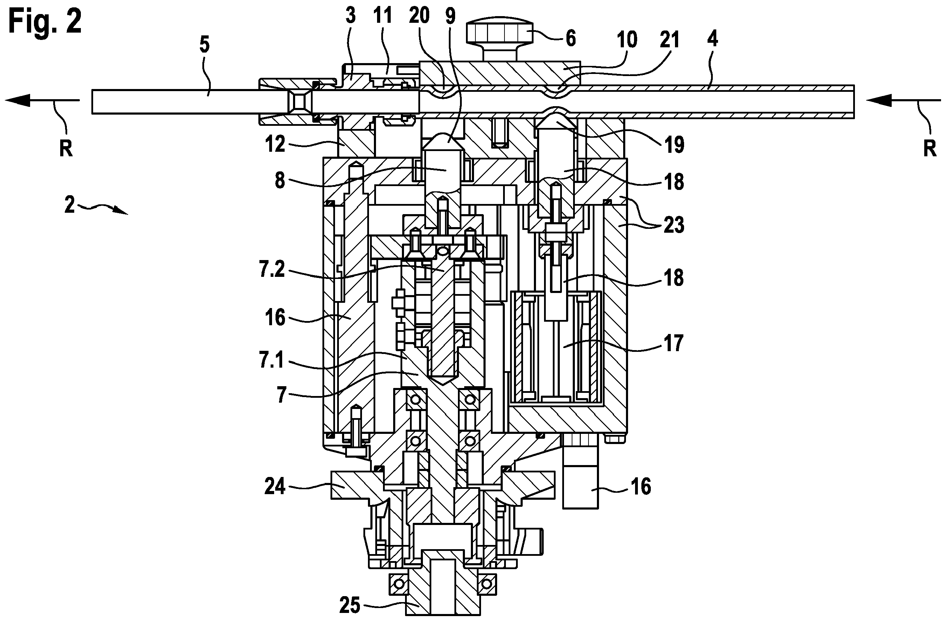

Figur 1 zeigt eine schematische Ansicht einer Fördervorrichtung einer ersten Ausführungsform und Figur 2 zeigt einen schematischen Schnitt durch die Fördervorrichtung aus Figur 1.

Die Fördervorrichtung 2 weist ein Drosselventil 3, einen an das Drosselventil 3 angeschlossenen Schlauch 4 und eine mittels eines elektrischen Antriebs antreibbare Hubvorrichtung 7 auf. Die Hubvorrichtung 7 weist ein Hubmittel 8 in Form eines mechanischen Stößels auf. Das Hubmittel 8 dient wenigstens zur teilweisen oder vollständigen Quetschung des Schlauchs 4. Vorzugsweise ist Hubvorrichtung 7 als ein Ventil oder eine Schlauchquetsche ausgebildet. Die Fördervorrichtung 2 kann als ein Adapterkopf für eine Füllstation verstanden werden.

An einem ersten Ende des Drosselventils 3 ist der Schlauch 4 angeschlossen. An einem zweiten Ende des Drosselventils 3 ist ein zweiter Schlauch 5 angeschlossen. Das Füllgut kann entsprechend der Förderrichtung R durch den Schlauch 4, die Drossel 3 und den zweiten Schlauch 5 gefördert werden.

Das Drosselventil 3 ist mittels einer Haltevorrichtung 11, 12 an der klappbaren Verschlusskappe 10 und am Gehäuse der Fördervorrichtung 2 befestigt. Ein erster Teil 11 der Haltevorrichtung ist als zwei längliche Auskragungen ausgebildet, die sich von der Verschlusskappe 10 in Richtung zu einem ersten Teilbereich des Drosselventils 3 erstrecken. Ein zweiter Teil 12 der Haltevorrichtung ist an einem Gehäuse 23 der Fördervorrichtung 2 angeordnet. Der zweite Teil 12 weist eine Aussparung zur Aufnahme eines zweiten Teilbereichs des Drosselventils 3 auf. Dabei ist das Drosselventil 3 zwischen den Teilen 11, 12 angeordnet und positioniert. Die Verschlusskappe 10 ist mittels eines Scharniers 13 am Gehäuse 23 der Fördervorrichtung 2 verkippbar gelagert. Damit lässt sich mittels einer einzigen Hand die Verschlusskappe 10 öffnen. Ferner ist ein Drehverschlussmittel 6 vorgesehen, welches wenigstens teilweise durch die Verschlusskappe 10 geführt ist. Nach einem erfolgtem Öffnen bzw. Wegklappen der Verschlusskappe kann der Schlauch 4, das Drosselventil 3 und/oder der zweite Schlauch 5 entnommen werden.

Das Hubmittel 8 weist ein erstes Quetschmittel 9 auf. Der Schlauch 4 ist zwischen dem ersten Quetschmittel 9 und der klappbaren Verschlusskappe 10 der Fördervorrichtung 2 angeordnet. Das erste Quetschmittel 9 weist eine Kante zur Quetschung des Schlauches 4 auf. Das erste Quetschmittel ist als ein Keil ausgebildet. An der Verschlusskappe 10 ist eine erste Erhöhung 20 vorgesehen. Die erste Erhöhung 20 ist gegenüber vom ersten Quetschmittel 9 verschlusskappenunterseitig angeordnet. Somit ist eine Quetschung des Schlauchs 4 auf einfache Weise ausführen.

Außerdem weist die Fördervorrichtung 2 einen Zwangsschließmechanismus 17 auf. Der Zwangsschließmechanismus 17 weist ein Hubmittel 18 in Form eines mechanischen Stößels auf. Das Hubmittel weist ein zweites Quetschmittel 19 auf. Der Schlauch 4 ist zwischen dem zweiten Quetschmittel 19 und der klappbaren Verschlusskappe 10 der Fördervorrichtung 2 angeordnet. Das zweite Quetschmittel 19 weist eine Kante zur Quetschung des Schlauchs 4 auf. Das zweite Quetschmittel 19 ist als ein Keil ausgebildet. An der Verschlusskappe 10 ist eine zweite Erhöhung 21 vorgesehen. Die zweite Erhöhung 20 ist gegenüber vom zweiten Quetschmittel 19 verschlusskappenunterseitig angeordnet. Somit ist eine Quetschung des Schlauchs 4 auf einfache Weise ausführen. Der Zwangsschließmechanismus 17 ist über einen Steuerluftanschluss 16 mit Druckluft versorgbar.

Wie oben bereits erwähnt, weist die Füllstation 1 die mittels des elektrischen Antriebs 40 antreibbare Welle 31 auf. Die Welle 31 ist dabei an einem ersten Ende an die Hubvorrichtung 7 und an einem zweiten Ende am elektrischen Antrieb 40 angeschlossen ist.

Die Hubvorrichtung 7 weist einen Spindeltrieb 7.1, 7.2 zur Verlagerung des Hubelements 8 der Hubvorrichtung 7 auf. Mittels des Spindeltriebs 7.1, 7.2, bestehend aus Spindelmutter 7.1 und Kugelumlaufspindel 7.2 kann die vom elektrischen Antrieb erzeugte Drehbewegung in eine Linearbewegung umgewandelt werden. Hierdurch ist eine axiale Verlagerung des Hubmittels 8 mittels des Spindeltriebs 7.1, 7.2 erreichbar.

Die Fördervorrichtung 2 weist drei Führungswellen 16 auf. Die Führungswellen 16 dienen der Stabilität und vermeiden eine Verdrehung innerhalb der Fördervorrichtung 2 während des Betriebs der Fördervorrichtung 2.

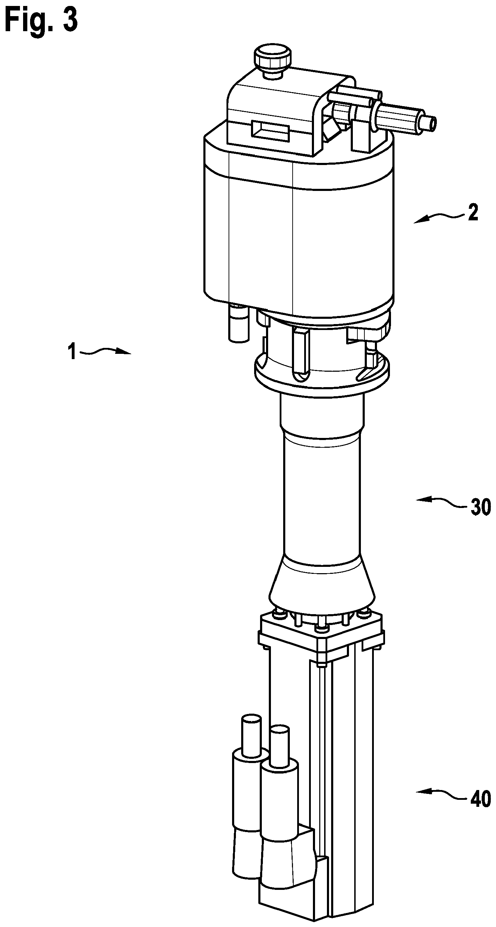

Figur 3 zeigt eine schematische Ansicht einer erfindungsgemäßen Ausführungsform einer Füllstation mit der Fördervorrichtung gemäß den Figuren 1 und 2. Die Füllstation 1 dient dem Fördern von Füllgut. Die Füllstation 1 weist die Fördervorrichtung 2, ein die Fördervorrichtung 2 aufnehmendes Wellengehäuse 30, die als Tragkonstruktion verstanden werden kann sowie einen am Wellengehäuse 30 angeordneten elektrischen Antrieb 40 auf. Die Fördervorrichtung 2 ist austauschbar und kann im Bedarfsfall ausgewechselt bzw. ausgetauscht werden.

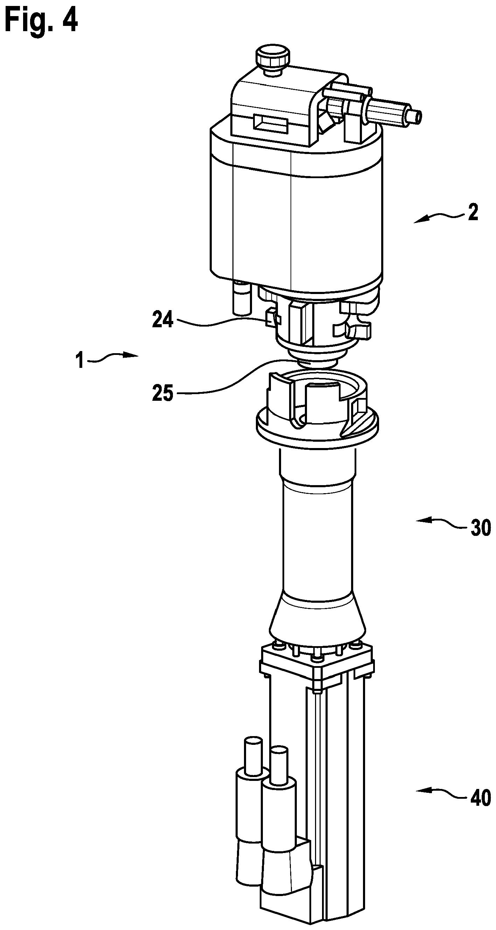

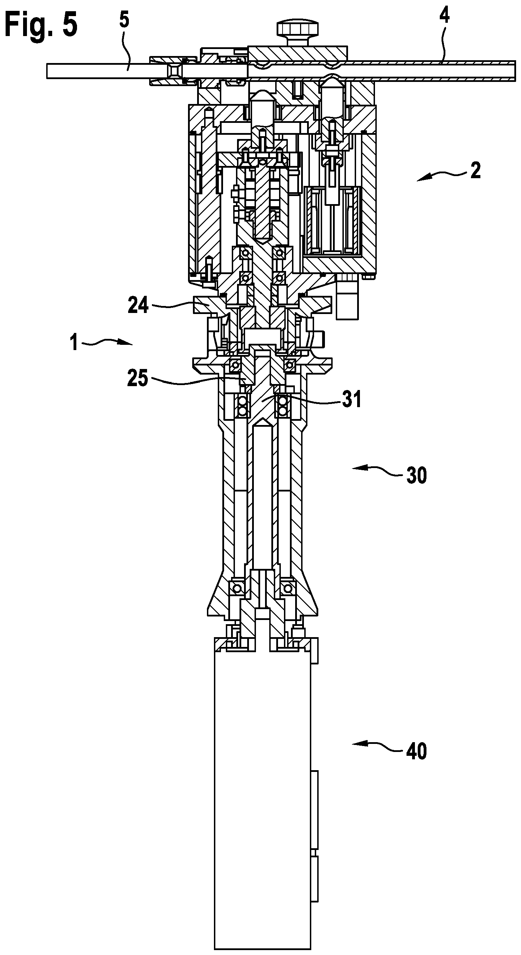

Figur 4 zeigt eine weitere schematische Ansicht durch die Ausführungsform aus Figur 3 und Figur 5 zeigt einen schematischen Schnitt durch die Ausführungsform aus Figur 3.

Die Fördervorrichtung 2 weist das Schnellverschlussmittel 24, das als Adaptierung verstanden werden kann, an das Wellengehäuse 30 auf. Ferner weist die Fördervorrichtung 2 zusätzlich die Kupplung 25, insbesondere Motorkupplung, zur Verbindung mit einer Welle 31 auf, die vom Wellengehäuse 30 aufgenommen ist. Durch das Vorsehen des Schnellverschlussmittels 24 ist die Fördervorrichtung 2 auf einfache Weise mit dem Wellengehäuse 30 verbindbar. Durch das Vorsehen der Kupplung 25 ist die innerhalb der Fördervorrichtung 2 angeordnete Hubvorrichtung 7 auf einfache Weise mit der Welle 31 verbindbar.

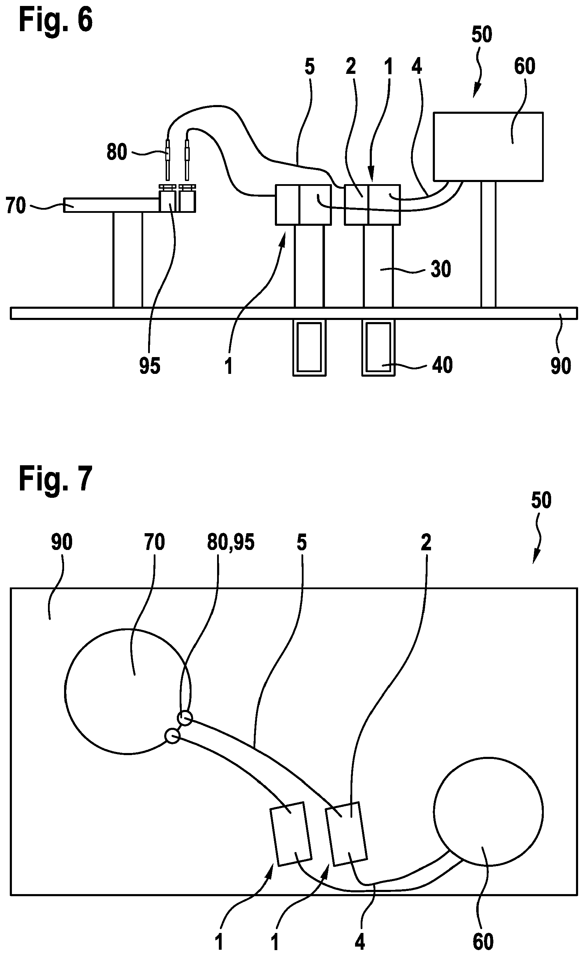

Figur 6 zeigt eine schematische Ansicht einer weiteren erfindungsgemäßen Ausführungsform einer Abfüllanlage mit einer Füllstation gemäß den Figuren 3 bis 5. Die Abfüllanlage 50 weist zwei Füllstationen 1 auf, wie oben bereits beschrieben, und Figur 7 zeigt eine schematische Draufsicht auf die Abfüllanlage 45 aus Figur 6.

Ein erstes Ende des Schlauchs 4 ist an einem Füllgutreservoir 60 angeschlossen. Im Füllgutreservoir 60 befindet sich Füllgut. Der Schlauch 4 ist an seinem zweiten Ende an die Drossel (nicht gezeigt) angeschlossen. Damit lässt sich Füllgut vom Füllgutreservoir 60 auf einfache Weise in Richtung zur Fördervorrichtung 2 fördern.

Ein erstes Ende des zweiten Schlauches 5 ist an die Drossel (nicht gezeigt) angeschlossen. Der zweite Schlauch 5 weist an seinem zweiten Ende eine Auslassöffnung 80 auf, insbesondere eine Füllnadel. Damit lässt sich Füllgut von der Fördervorrichtung 2 in zu befüllende Behältnisse 95, die auf einer Transportvorrichtung 70 transportierbar sind, auf einfache Weise fördern.

Die zwei Füllstationen 1, das Füllgutreservoir 60 sowie die Transportvorrichtung 70 sind auf einer Grundplatte 90 der Abfüllanlage 50 angeordnet. An einer Unterseite der Grundplatte 90 sind zwei elektrische Antriebe 40 angeordnet.

## Patentansprüche

1. Füllstation (1) zum Fördern von Füllgut von einem Reservoir (50) zu einer Ausgabe (80) durch einen Schlauch (4), aufweisend den Schlauch (4), eine den Schlauch (4) aufnehmende und austauschbare Fördervorrichtung (2), eine innerhalb eines Wellengehäuses (30) angeordnete Welle (31) und einen elektrischen Antrieb (40), wobei die Welle (31) mittels des elektrischen Antriebs (40) antreibbar ist und wobei die Welle (31) mit einem ersten Ende mit der Fördervorrichtung (2) verbindbar ist.

2. Füllstation (1) nach Anspruch 1, wobei die Fördervorrichtung (2) zur Verbindung mit dem Wellengehäuse (30) und zur Kopplung mit der Welle (31) ein Schnellverschlussmittel (24) aufweist.

3. Füllstation (1) nach einem der vorherstehenden Ansprüche, wobei die Fördervorrichtung (2) eine mittels der Welle (31) antreibbare Hubvorrichtung (7) und eine klappbare Verschlusskappe (10) aufweist.

4. Füllstation (1) nach Anspruch 3, wobei die Hubvorrichtung (7) ein Hubmittel (8) wenigstens zur teilweisen oder vollständigen Quetschung des Schlauchs (4) aufweist und wobei der Schlauch (4) zwischen dem Hubmittel (8) und der klappbaren Verschlusskappe (10) der Fördervorrichtung (2) angeordnet ist.

5. Füllstation (1) nach Anspruch 4, wobei die Hubvorrichtung (8) einen Spindeltrieb (7.1, 7.2) zur Verlagerung des Hubmittels (8) aufweist.

6. Füllstation (1) nach einem der vorherstehenden Ansprüche, wobei die Fördervorrichtung (2) ein Drosselventil (3) aufweist, das an einer Seite des Schlauchs (4) angeschlossen ist.

7. Füllstation (1) nach Anspruch 6, wobei das Drosselventil (3) mittels einer Haltevorrichtung (11, 12) an der klappbaren Verschlusskappe (10) und am Gehäuse der Fördervorrichtung (2) befestigbar ist.

8. Füllstation (1) nach einem der vorherstehenden Ansprüche, wobei die Fördervorrichtung (2) einen Zwangsschließmechanismus (17) aufweist.

9. Füllstation (1) nach einem der vorherstehenden Ansprüche, wobei die Füllstation (1) als Kombifüllstation ausgebildet ist.

10. Abfüllanlage (50) aufweisend wenigstens eine Füllstation (1) nach einem der vorherstehenden Ansprüche.
